# EUROPEAN PATENT APPLICATION

(11) **EP 3 979 192 A1**
(43) Date of publication of application: **06.04.2022**
(21) Application number: 20199407.6
(22) Date of filing: 30.09.2020
(51) Int. Cl.: G06T 7/00, G06T 7/11, A61B 5/00, A61B 6/00

(54) **PAEDIATRIC BONE AGE ASSESSMENT OF X-RAY IMAGES BASED ON DETECTION OF OSSIFICATION REGIONS**

(71) Applicant: AGFA NV, 2640 Mortsel (BE)
(72) Inventor: DANUDIBROTO, Adriyana, 2640 Mortsel (BE); VACA CERDA, Esteban Alejandro, 2640 Mortsel (BE)
(74) Representative: Pieters, Dirk Paul Oscar

(57) **Abstract**

The present invention relates to a method to accurately assess the bone age of paediatric subjects, based on an automatic deep-learning approach to perform bone age assessment of hand X-Ray images. The approach consists of a convolutional neural network which performs ossification region detection on the hand X-ray image and bone age prediction of each detected region in one processing pipeline. The model may combine the gender metadata with the image information to support the prediction of the bone age of the patient.

## Description

### Technical Field

The present invention is situated in the field of medical image processing, and is based on the application of convolutional neural networks in this field. More specifically, portions of this disclosure relate to determining skeletal maturity of a patient by processing radiograph images.

### Background of the invention

Bone age assessment (BAA) is a measure of bone development or skeletal maturity. It takes into account the size, shape and degree of ossification (mineralization) of the bones, which estimates the proximity of the bones to full maturity. During child development, multiple factors may cause a difference between the chronological age and the bone age, including, among others: paediatric syndromes, endocrine disorders, growth problems, poor nutrition or genetic diseases.

In paediatrics, the use of bone age assessment has a broader application than only the detection of growth disorders, such as for instance the early discovery of elite athletes, or a contribution to civil registration programmes. A reliable bone age assessment can enable the reliable determination of the age of children that do not have identification or birth registration documents. The technique could support these children to claim their rights. In the last years, bone age assessment has also been useful in the case of children seeking asylum, where it provides the basis to allow the correct allocation of humanitarian help resources.

The predominant clinical procedure of bone age assessment is the atlas by Greulich and Pyle (G&P atlas, 1959), which uses an X-ray of the left hand as the basis for its estimations. The method consists of visual observation of the skeletal maturation of the whole hand. It compares a subject radiograph with an atlas which is a collection of hand radiographies of children gathered in the United States between 1931 and 1942; the physician assigns the bone age according to the most similar image in the atlas. Despite the popularity of this method, it does not set a standard procedure to assess the bone age reliably, and it depends on the expertise of the physicians. Thus it may be biased by human interpretation.

In an attempt to overcome the drawbacks of the G&P atlas a different method was developed by Tanner-Whitehouse (Tanner-Whitehouse method, 2001). Initially designed in 1975, the TW-method analyses particular bones of the hand including the radius, ulna, carpal bones of the wrist, metacarpals, and phalanges. The method takes into account certain metadata like the gender and the race of the subject. The study presented by Zhang et al. (Zhang, A., Sayre, J.W., Vachon, L., Liu, B.J., Huang, H., 2009. Racial differences in growth patterns of children assessed on the basis of bone age. Radiology 250, 228-235) showed that there exist differences in growth patterns according to the ethnic and race of the subjects; such differences directly affect the bone age assessment. Nevertheless, the G&P atlas did not acknowledge this fact.

Besides its application in predicting the bone age of a subject, the Tanner-Whitehouse method also aims to predict the adult body height taking into account some additional parameters such as the current subject height, the chronological age and some height coefficients.

The Tanner-Whitehouse method partitions a radiograph into several regions of interest (ROIs) and assigns a maturity stage per ROI with a letter between (A, B, ..., I). At the same time, it takes race and gender information, and some age coefficients into account, and it converts the marked stages into a numerical score. The resulting bone age is the addition of the numeric scores of the ROIs.

Regardless of the wide acceptance of the G&P atlas and the Tanner-Whitehouse method, their practical application requires thorough physician analysis and have a high dependency on the expertise and experience of the radiologists putting the methods to practice. In a practical setting, the required training to achieve a certain level of experience is very time consuming. These facts, coupled with the growing demand for this type of analysis in the immigration context and paediatrics, make these methods susceptible to high inter and intra-rater variability.

In this context, technological advances in computer vision have been used to try to reduce the rating variability in bone age assessment. Most of these advances are based on image processing technique aiming to replicate the clinical approach of G&P and the Tanner-Whitehouse methods. One approach to facilitate bone age assessment was the digital atlas of skeletal maturity presented by Gilsanz and Ratib (2005), which introduced a portable computer containing the G&P atlas to help the radiologists perform bone age assessment manually. Towards automating bone age assessment, several strategies have been developed in recent years, most of them inspired by the Tanner-Whitehouse method. Indeed, the sequential structure of the Tanner-Whitehouse method based on the score of various ROIs enables the development of automated algorithms primarily focused on the localisation of the ROIs and performing feature extraction to predict the skeletal maturity of the subject.

The evolution of machine learning and deep learning has demonstrated performance improvements in a wide array of medical applications such as computer assisted diagnosis, image segmentation, and object localisation. In this context, numerous proposals have been developed to perform automatic bone age assessment with deep learning. The approaches range from the direct application of convolutional neural networks (CNNs) with a regression head to ensembles of predictions of the ROIs of the Tanner-Whitehouse method.

### Summary of invention

The present invention provides a method for determining the bone age of a paediatric subject, comprising the steps of receiving, by a communication module of at least one computer, a target image that is a specific medical image of a complete hand, extracting image features from multiple scale level, by means of applying a feature extractor and combining the features from the different scale levels in a combination of top-down and bottom-up approach for further processing, localising epiphyseal growth regions (or ossification regions) in said target image, by means of applying a trained object detection net that uses a combination of image information from multiple scale levels extracted by the aforementioned feature extractor as input, labelling said localised epiphyseal growth regions (or ossification regions) in said target image, by means of applying a trained classification net that uses a combination of image information from multiple scale levels extracted by the aforementioned feature extractor as input, determining the bone age of said localised epiphyseal growth regions by means of applying a trained regression net that uses a combination of image information from multiple scale levels extracted by the aforementioned feature extractor as input, characterized in that the determination of the bone age per said localised epiphyseal growth region, the localization and the labelling of the epiphyseal growth regions are performed by a single processing pipeline.

In the context of the invention, the target image considered is an X-ray radiograph of a patient's hand taken in a PA-view (posterior-anterior view) that comprises the image of the wrist.

The epiphyseal plate, the area of growth composed of four zones, is where cartilage is formed on the epiphyseal side while cartilage is ossified on the diaphyseal side, thereby lengthening the bone. Each of the four zones has a role in the proliferation, maturation, and calcification of bone cells that are added to the diaphysis. The longitudinal growth of long bones continues until early adulthood at which time the chondrocytes in the epiphyseal plate stop proliferating and the epiphyseal plate transforms into the epiphyseal line as bone replaces the cartilage. Bones can increase in diameter even after longitudinal growth has stopped.

The epiphyseal growth regions of the hand can be separated in different groups; they include four regions between the distal and intermediate phalanges (DIP), five spaces between the intermediate and proximal phalanges (PIP), the five regions between the proximal phalanges and the metacarpals (MCP) the carpal bones (Wrist), the radius and ulna.

In the context of this application, an epiphyseal growth region is a zone in the skeleton of a patient where there is an ossification region.

Object detection is a computer vision technique that allows us to identify and locate objects in an image or video. With this kind of identification and localization, object detection can be used to count objects in a scene and determine and track their precise locations, all while accurately labelling them. In artificial intelligence, an object detection model (that may be implemented as a object detection network) is trained on a specific task that it is designed for. The object detection network (or net) is trained by feeding it correctly labelled training data.

A trained object detection net is thus a fully functional network that has already been trained on a specific task it has been designed for. In the context of this patent application, the trained object detection net has been trained to identify and detect the presence of epiphyseal growth regions. The object detection net provides as its output a location marker that indicates the presence of such a growth region in the original input image.

A classification model tries to draw some conclusions from the input values given for training. It will predict the class labels or categories for the new data that is presented to it as input. In the context of this application, the trained classification net will determine the type of epiphyseal growth region that was detected by the object detection model above. The outcome of the application of this classification model on a processed image allows the labelling of the detected growth region with the appropriate growth region type information, such that a user can easily review and validate the results of the algorithm.

A pyramid representation is a type of multi-scale signal representation developed for computer vision, image processing and signal processing applications, in which a signal or an image is subject to repeated smoothing by a selected filtering kernel and subsampling. Multiple scale levels are predecessors to multiresolution analysis. In the context of this invention, the multiple scale levels are calculated from the original input image and serve subsequently as input for the trained object detection and classification models. Furthermore, the filtering and subsampling process in the context of this invention is an inherent part of the object detection model.

The invention is advantageous in that it performs different steps that are contributing to a high performance bone age assessment at the same time. Even some of the intermediary results that contribute to the bone age assessment, namely the detection and identification (labelling) of the epiphysial growth regions, are performed in a single pipeline which increases the robustness (accuracy) and speed of the image pipeline in comparison with a sequential approach. Moreover, the entire pipeline can be trained on the same image dataset given the relevant bone age and ROI labels.

The embodiments of the systems and methods described herein may be implemented in hardware or software, or a combination of both. However, preferably, these embodiments are implemented in computer programs executing on programmable computers each comprising at least one module component which comprises at least one processor (e.g. a microprocessor), a data storage system (including volatile and non-volatile memory and/or storage elements), at least one input device, and at least one output device. For example and without limitation, the programmable computers (referred to here as computer systems) may be a personal computer, laptop, personal data assistant, and cellular telephone, smart-phone device, tablet computer, and/or wireless device. Program code is applied to input data to perform the functions described herein and generate output information. The output information is applied to one or more output devices, in known fashion.

Each program is preferably implemented in a high level procedural or object oriented programming and/or scripting language to communicate with a computer system. However, the programs can be implemented in assembly or machine language, if desired. In any case, the language may be a compiled or interpreted language. Each such computer program is preferably stored on a storage media or a device (e.g. ROM or magnetic diskette) readable by a general or special purpose programmable computer, for configuring and operating the computer when the storage media or device is read by the computer to perform the procedures described herein. The subject system may also be considered to be implemented as a computer-readable storage medium, configured with a computer program, where the storage medium so configured causes a computer to operate in a specific and predefined manner to perform the functions described herein.

Specific examples and preferred embodiments are set out in the dependent claims. Further advantages and embodiments of the present invention will become apparent from the following description and drawings.

### Brief description of drawings

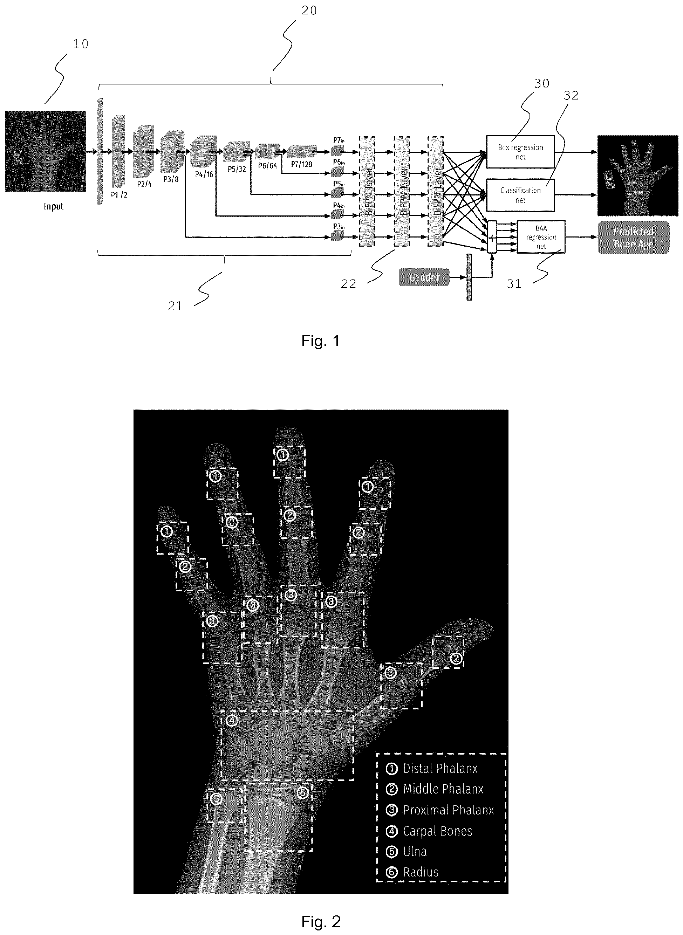

Fig. 1 gives a schematic overview of the image processing pipeline that is applied in the method of the invention. An input image of a hand [10] is presented to one of the EfficientDet models [20] with EfficientNet architecture [21] as a feature extractor, followed by a BiFPN [22] that fuses the features efficiently, whose data is fed into 2 regressions net and one classification net. The first regression net [30] is a box regression net to localise the epiphyseal growth regions, while the second regression net [31] is the bone age assessment (BAA) regression net. The classification net [32] is the classification net that classifies the type of the localised epiphyseal growth region detected by the box regression net.
Fig. 2 depicts the regions of interest identified in the Tanner-Whitehouse methods to assess the bone age. Different regions of interest in the X-ray image of a hand are indicated with numbers [1, 2, 3, 4, 5, 6].

### Description of embodiments

In the following detailed description, reference is made in sufficient detail to the above referenced drawings, allowing those skilled in the art to practice the embodiments explained below.

In the following section, the proposed strategy for bone age assessment is described; it was found that the application of cutting edge deep learning architectures used for classification and object detection of conventional images can improve the accuracy of bone age assessment.

Different approaches for bone age assessment have been evaluated not only by using the whole image but also by using selected information of different anatomical structures that are present in an X-ray image from a hand. The design of our algorithm is based on the identification and analysis of the epiphyseal growth regions of the Tanner-Whitehouse method.

In contrast to previous studies based on multiple stage algorithms, the herein introduced approach performs bone age assessment in a single step and explores the different epiphyseal growth regions in the X-ray images simultaneously.

The method simultaneously localises the epiphyseal growth regions, also called ossification regions, and labels them. The epiphyseal growth regions can be separated in different groups; they include four regions between the distal and intermediate phalanges (DIP), five spaces between the intermediate and proximal phalanges (PIP), the five regions between the proximal phalanges and the metacarpals (MCP) the carpal bones (Wrist), the radius and ulna. The labelling of the growth regions according to the different groups is performed simultaneously with the localisation of them.

The localisation of these epiphyseal growth regions can be achieved by means of deep-learning object detection algorithms. In recent years, new approaches have been proposed to perform object detection. EfficientDet by Tan et al. [Tan, M., Pang, R., Le, Q.V., 2019. Efficientdet: Scalable and efficient object detection. arXiv preprint arXiv:1911.09070] achieved the state of the art performance in the Common Objects in Context (COCO) dataset released by Lin et al. (2014), their strategy uses a one stage detector with efficient multi-scale feature fusion using bi-directional feature pyramid network (BiFPN) that feeds into a classification and a box regression subnetwork. BiFPN efficiently fuses the features from multi-scale level in a combination of top-down and bottom-up direction. Another unique strategy used by EfficientDet is the compound scaling method that uniformly scales the size of the image input and the depth and width of the feature extractor, the BiFPN and the box regression and classification net. A regression head in the EfficientDet architecture was added to predict the bone age of the subjects.

A next step is the determination of the bone age estimation per epiphyseal growth region that was detected by the trained object detection net. The results will then be consolidated for each input image to obtain a bone age assessment for the paediatric subject.

The method allows to render its intermediary results (being the object location detection of the epiphyseal growth regions, the determination of the type of the epiphyseal growth regions according to the Tanner-Whitehouse method, and the bone age assessment for each epiphyseal growth region) as an overlay on the original input X-ray image.

According to the Tanner-Whitehouse methods, gender is crucial metadata to be considered for bone age assessment. Therefore, a fully connected layer was used to merge gender information with the features extracted from the image. The merging of said gender information may be performed by means of concatenating or summing the corresponding features. Other metadata that could also be considered to be included in the prediction of the bone age includes chronological age, race, and socio-economic background.

The final proposed model contains three prediction heads including box regression (to perform the localisation of the epiphyseal growth regions), classification of the regions (to label the growth regions) and a bone age assessment head. The presented model can be trained in an end to end way using the labels of the three tasks at the same time.

An initial and optional step comprises the segmentation of the hand that may be performed as a first preprocessing step to isolating the relevant image information from the irrelevant image background.

## Claims

1. Method for determining the bone age of a paediatric subject, comprising the steps of:
- receiving, by a communication module of at least one computer, a target image that is a specific medical image of a complete hand [10],
- extracting image features from multiple scale levels, by means of applying a feature extractor [21] and combining the features from the different scale levels in a combination of top-down and bottom-up approach for further processing,
- localising epiphyseal growth regions [1-6] in said target image, by means of applying a trained object detection net [30] that uses a combination of image information from multiple scale levels extracted by the aforementioned feature extractor as input,
- labelling said localised epiphyseal growth regions in said target image, by means of applying a trained classification net [32] that uses a combination of image information from multiple scale levels extracted by the aforementioned feature extractor as input,
- determining the bone age of said localised epiphyseal growth regions by means of applying a trained regression net [31] that uses a combination of image information from multiple scale levels extracted by the aforementioned feature extractor as input,
**characterized in that**
- the determination of the bone age per said localised epiphyseal growth region, the localization and the labelling of the epiphyseal growth regions are performed by a single processing pipeline.

2. Method according to Claim 1, wherein a bone age assessment for the paediatric subject is performed based on a consolidation step that is based on the determinations of the bone age of said localised epiphyseal growth regions.

3. Method according to Claim 1 and 2, wherein a hand segmentation preprocessing step is performed prior to using the image information in the method to exclude non-clinical information from the image.

4. Method according to Claim 1 and 2, wherein gender information is merged with said image information from said multiple scale levels before it is used as input of said trained object detection and classification nets.

5. Method according to Claim 4, wherein said merging of said gender information is performed by means of concatenating or summing corresponding features.

6. Method according to Claim 1 and 2, wherein socio-economical information or racial information is merged with said image information from said multiple scale levels before it is used as input of said trained object detection and classification nets.
